# EUROPÄISCHE PATENTANMELDUNG

(11) **EP 1 468 998 A1**
(43) Veröffentlichungstag der Anmeldung: **20.10.2004**
(21) Anmeldenummer: 04016877.5
(22) Anmeldetag: 28.09.2001
(51) Int. Cl.: C07D 451/10, A61K 31/46, A61P 43/00

(54) **Kristallines Monohydrat von Tiotropiumbromid und Verfahren zu dessen Herstellung.**

(30) Priorität: 12.10.2000 DE 10050621
(62) Teilanmeldung aus: 01983510.7
(71) Anmelder: Boehringer Ingelheim Pharma GmbH & Co. KG, 55216 Ingelheim am Rhein (DE)
(72) Erfinder: Banholzer, Ralf, 70597 Stuttgart (DE); Graulich, Manfred, 55425 Waldalgesheim (DE); Kullina, Christian, 88448 Attenweiler (DE); Mathes, Andreas, 55437 Ockenheim (DE); Meissner, Helmut, 55218 Ingelheim (DE); Sieger, Peter, 88441 Mittelbiberach (DE); Specht, Peter, 55437 Ober-Hilbersheim (DE); Trunk, Michael, 55218 Ingelheim (DE)

(57) **Zusammenfassung**

Die Erfindung betrifft ein kristallines Monohydrat von (1α,2β,4β,5α,7β)-7-[(hydroxydi-2-thienylacetyl)oxy]-9,9-dimethyl-3-oxa-9-azoniatricyclo[3.3.1.0^{2,4}]nonane-bromid, Verfahren zu dessen Herstellung, sowie dessen Verwendung zur Herstellung eines Arzneimittels, insbesondere zur Herstellung eines Arzneimittels mit anticholinerger Wirkung.

## Beschreibung

Die Erfindung betrifft ein kristallines Monohydrat von (1α,2β,4β,5α,7β)-7-[(hydroxydi-2-thienylacetyl)oxy]-9,9-dimethyl-3-oxa-9-azoniatricyclo[3.3.1.0^{2,4}]nonane-bromid, Verfahren zu dessen Herstellung, sowie dessen Verwendung zur Herstellung eines Arzneimittels, insbesondere zur Herstellung eines Arzneimittels mit anticholinerger Wirkung.

### Hintergrund der Erfindung

Die Verbindung (1α,2β,4β,5α,7β)-7-[(hydroxydi-2-thienylacetyl)oxy]-9,9-dimethyl-3-oxa-9-azoniatricyclo[3.3.1.0^{2,4}]nonane-bromid, ist aus der Europäischen Patentanmeldung EP 418 716 A1 bekannt und weist die folgende chemische Struktur auf:

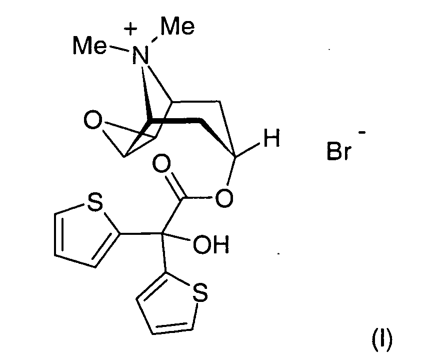

Die Verbindung besitzt wertvolle pharmakologische Eigenschaften und ist unter dem Namen Tiotropiumbromid (BA679) bekannt. Tiotropiumbromid stellt ein hochwirksames Anticholinergikum dar und kann deshalb bei der Therapie von Asthma oder COPD (chronic obstructive pulmonary disease = chronisch obstruktive Lungenerkrankung) einen therapeutischen Nutzen entfalten.

Die Applikation von Tiotropiumbromid erfolgt vorzugsweise auf inhalativem Wege. Hierbei können geeignete Inhaltionspulver, die in geeignete Kapseln (Inhaletten) abgefüllt mittels entsprechender Pulverinhalatoren appliziert werden, zum Einsatz kommen. Alternativ dazu kann eine inhalative Anwendung auch durch Applikation geeigneter Inhalationsaerosole erfolgen. Hierzu zählen auch pulverförmige Inhalationsaerosole, die beispielsweise HFA134a, HFA227 oder deren Gemisch als Treibgas enthalten.

Die richtige Herstellung der vorgenannten, zur inhalativen Verabreichung eines Arzneiwirkstoffes verwendbaren Zusammensetzungen stützt sich auf verschiedene Parameter, die mit der Beschaffenheit des Arzneiwirkstoffes selbst verbunden sind. Ohne Einschränkung darauf sind Beispiele dieser Parameter die Wirkstabilität des Ausgangsstoffes unter verschiedenen Umgebungsbedingungen, die Stabilität im Verlauf der Herstellung der pharmazeutischen Formulierung sowie die Stabilität in den Endzusammensetzungen des Arzneimittels. Der zur Herstellung der vorgenannten Arzneimittelzusammensetzungen verwendete Arzneiwirkstoff sollte so rein wie möglich sein und seine Stabilität bei Langzeitlagerung muß unter verschiedenen Umgebungsbedingungen gewährleistet sein. Dies ist zwingend erforderlich, um zu verhindern, daß Arzneimittelzusammensetzungen Verwendung finden, in denen neben tatsächlichem Wirkstoff beispielsweise Abbauprodukte desselben enthalten sind. In einem solchen Fall könnte ein in Kapseln vorgefundener Gehalt an Wirkstoff niedriger sein als spezifiziert.
Die Absorption von Feuchtigkeit vermindert den Gehalt an Arzneiwirkstoff wegen der durch die Wasseraufnahme verursachten Gewichtszunahme. Zur Aufnahme von Feuchtigkeit neigende Arzneimittel müssen während der Lagerung vor Feuchtigkeit geschützt werden, beispielsweise durch Zusatz von geeigneten Trockenmitteln oder durch Lagerung des Arzneimittels in einer vor Feuchtigkeit geschützten Umgebung. Zudem kann die Aufnahme von Feuchtigkeit den Gehalt an Arzneiwirkstoff während der Herstellung vermindern, wenn das Arzneimittel der Umgebung ohne jeglichen Schutz vor Feuchtigkeit ausgesetzt wird.

Eine gleichmäßige Verteilung des Arzneimittels in der Formulierung ist ein kritischer Faktor, insbesondere wenn eine niedrige Dosierung des Arzneimittels erforderlich ist. Um die gleichmäßige Verteilung sicherzustellen, kann man die Teilchengrösse des Wirkstoffes auf einen geeigneten Wert vermindern, beispielsweise durch Mahlen. Ein weiterer Aspekt, der bei inhalativ, mittels eines Pulvers zu applizierenden Wirkstoffen bedeutsam ist, ist durch den Umstand bedingt, daß nur Teilchen einer bestimmten Teilchengröße bei der Inhaltion in die Lunge gelangen. Die Teilchengröße dieser lungengängigen Partikel (inhalierbarer Anteil) liegt im Submicronbereich. Um Wirkstoffe mit entsprechender Teilchengröße zu erhalten, ist ebenfalls ein Mahlvorgang (sogenanntes Mikronisieren) erforderlich.
Da als Begleiterscheinung des Mahlens (bzw. Mikronisierens) trotz der beim Verfahrensablaufs erforderlichen harten Bedingungen ein Abbau des Arzneimittelwirkstoffs weitestgehend vermieden werden muß, stellt eine hohe Stabilität des Wirkstoffs gegenüber dem Mahlvorgang eine unabdingbare Notwendigkeit dar. Nur eine ausreichend große Stabilität des Wirkstoffs beim Mahlvorgang erlaubt die Herstellung einer homogenen Arzneimittelformulierung, in der stets in reproduzierbarer Art und Weise die festgelegte Menge an Wirkstoff enthalten ist.

Eine weitere Problematik die beim Mahlvorgang zur Herstellung der gewünschten Arzneimittelformulierung auftreten kann, ist die durch diesen Prozess erfolgende Energiezufuhr und die Belastung der Oberfläche der Kristalle. Dies kann unter Umständen zu polymorphen Veränderungen, zu einer Umwandlung zur amorphen Gestalt hin oder zu einer Änderung des Kristallgitters führen. Da für die pharmazeutische Qualität einer Arzneimittelformulierung stets dieselbe kristalline Morphologie des Wirkstoffs gewährleistet sein muß, sind auch vor diesem Hintergrund an die Stabilität und Eigenschaften des kristallinen Wirkstoffs erhöhte Anforderungen zu stellen.

Die Stabilität eines Arzneiwirkstoffes ist in Arzneimittelzusammensetzungen ferner wichtig zur Festlegung der Gültigkeitsdauer der Arzneimittelspezialität; diese Dauer ist diejenige, während der das Arzneimittel ohne irgendwelches Risiko verabreicht werden kann. Eine hohe Stabilität eines Arzneimittels in den vorgenannten Arzneimittelzusammensetzungen unter verschiedenen Lagerungsbedingungen stellt deshalb sowohl für die Patienten wie auch für den Hersteller einen weiteren Vorteil dar.

Neben den vorstehend angegebenen Erfordernissen ist generell zu berücksichtigen, dass jede Änderung des Feststoffzustandes eines Arzneimittels, welche dessen physikalische und chemische Stabilität verbessern kann, gegenüber weniger stabilen Formen desselben Arzneimittels einen erheblichen Vorteil ergibt.

Die Aufgabe der Erfindung besteht somit in der Bereitstellung einer neuen, stabilen Kristallform der Verbindung Tiotropiumbromid, die den vorstehend genannten hohen Anforderungen, die an einen Arzneimittelwirkstoff zu richten sind, genügen.

### Detaillierte Beschreibung der Erfindung

Es wurde gefunden, daß Tiotropiumbromid je nach Wahl der Bedingungen, die bei der Reinigung des nach der technischen Herstellung erhaltenen Rohprodukts angewendet werden können, in unterschiedlichen kristallinen Modifikationen anfällt.

Es wurde gefunden, daß diese unterschiedlichen Modifikationen maßgeblich durch Wahl der zur Kristallisation eingesetzten Lösemittel sowie durch Wahl der beim Kristallisationsprozess gewählten Verfahrensbedingungen gezielt erhalten werden können.

Es wurde überraschenderweise festgestellt, daß das Monohydrat des Tiotropiumbromids, welches durch die Wahl spezifischer Reaktionsbedingungen in kristalliner Form erhalten werden kann, den eingangs genannten hohen Anforderungen genügt und somit die der vorliegenden Erfindung zugrunde liegende Aufgabe löst. Dementsprechend betrifft die vorliegende Erfindung kristallines Tiotropiumbromid-Monohydrat.

Ein weiterer Aspekt der vorliegenden Erfindung betrifft ein Herstellverfahren von kristallinen Hydraten des Tiotropiumbromids. Dieses Herstellverfahren ist dadurch gekennzeichnet, daß Tiotropiumbromid, welches beispielsweise nach der in der EP 418 716 A1 offenbarten Herstellungsvorschrift erhalten worden ist, in Wasser aufgenommen wird, die erhaltene Mischung erwärmt wird und abschließend die Hydrate des Tiotropiumbromids unter langsamem Abkühlen kristallisiert werden. Die vorliegende Erfindung betrifft ferner kristalline Hydrate des Tiotropiumbromids, die durch vorstehende Vorgehensweise erhältlich sind.

Ein Aspekt der vorliegenden Erfindung betrifft ein Herstellverfahren von kristallinem Tiotropiumbromid-monohydrat, welches nachfolgend detaillierter beschrieben wird. Zur Herstellung des kristallinen Monohydrats gemäß der vorliegenden Erfindung ist es erforderlich, Tiotropiumbromid, welches beispielsweise nach der in der EP 418 716 A1 offenbarten Herstellungsvorschrift erhalten worden ist, in Wasser aufzunehmen, zu Erwärmen, eine Reinigung mit Aktivkohle durchzuführen und nach Abtrennen der Aktivkohle unter langsamem Abkühlen das Tiotropiumbromid-monohydrat langsam zu kristallisieren.
Erfindungsgemäß bevorzugt wird wie nachfolgend beschrieben vorgegangen.
In einem geeignet dimensionierten Reaktionsgefäß wird das Lösemittel mit Tiotropiumbromid, welches beispielsweise nach der in der EP 418 716 A1 offenbarten Herstellungsvorschrift erhalten worden ist, gemischt.
Pro Mol eingesetztes Tiotropiumbromid werden 0,4 bis 1,5 kg, bevorzugt 0,6 bis 1 kg, besonders bevorzugt ca. 0,8 kg Wasser als Lösemittel verwendet.
Die erhaltene Mischung wird unter Rühren erwärmt, vorzugsweise auf mehr als 50°C, besonders bevorzugt auf mehr als 60°C. Die maximal wählbare Temperatur bestimmt sich durch den Siedepunkt des verwendeten Lösemittels Wasser. Vorzugsweise wird die Mischung auf einen Bereich von 80-90°C erhitzt.
In diese Lösung wird Aktivkohle, trocken oder wasserfeucht, eingebracht. Bevorzugt werden pro Mol eingesetztes Tiotropiumbromid 10 bis 50 g, besonders bevorzugt 15 bis 35 g, höchst bevorzugt etwa 25 g Aktivkohle eingesetzt. Gegebenenfalls wird die Aktivkohle vor Einbringen in die Tiotropiumbromid-haltige Lösung in Wasser aufgeschlämmt. Pro Mol eingesetztes Tiotropiumbromid werden zum Aufschlämmen der Aktivkohle 70 bis 200 g, bevorzugt 100 bis 160 g, besonders bevorzugt ca. 135 g Wasser verwendet. Wird die Aktivkohle vor Einbringen in die Tiotropiumbromid-haltige Lösung zuvor in Wasser aufgeschlämmt, empfiehlt es sich, mit der gleichen Menge Wasser nachzuspülen.
Bei konstanter Temperatur wird nach erfolgter Aktivkohlezugabe zwischen 5 bis 60 Minuten, bevorzugt zwischen 10 und 30 Minuten, besonders bevorzugt etwa 15 Minuten weitergerührt und die erhaltene Mischung filtriert, um die Aktivkohle zu entfernen. Der Filter wird anschließend mit Wasser nachgespült. Hierfür werden pro Mol eingesetztes Tiotropiumbromid 140 bis 400 g, bevorzugt 200 bis 320 g, höchst bevorzugt ca. 270 g Wasser verwendet.
Das Filtrat wird anschließend langsam abgekühlt, vorzugsweise auf eine Temperatur von 20-25°C. Die Abkühlung wird vorzugsweise mit einer Abklühlrate von 1 bis 10°C pro 10 bis 30 Minuten, bevorzugt von 2 bis 8°C pro 10 bis 30 Minuten, besonders bevorzugt von 3 bis 5°C pro 10 bis 20 Minuten, höchst bevorzugt von 3 bis 5°C pro ca. 20 Minuten durchgeführt. Gegebenenfalls kann sich nach dem Abkühlen auf 20 bis 25°C eine weitere Abkühlung auf unter 20°C, besonders bevorzugt auf 10 bis 15°C anschließen.
Nach erfolgter Abkühlung wird zwischen 20 Minuten und 3 Stunden, vorzugsweise zwischen 40 Minuten und 2 Stunden, besonders bevorzugt etwa eine Stunde zur Vervollständigung der Kristallisation nachgerührt.
Die entstandenen Kristalle werden abschließend durch Filtrieren oder Absaugen des Lösemittels isoliert. Sollte es erforderlich sein, die erhaltenen Kristalle einem weiteren Waschschritt zu unterwerfen, empfiehlt es sich als Waschlösemittel Wasser oder Aceton zu verwenden. Pro Mol eingesetztes Tiotropiumbromid können zum Waschen der erhaltenen Tiotropiumbromid-monohydrat-Kristalle 0,1 bis 1,0 L, bevorzugt 0,2 bis 0,5 L, besonders bevorzugt etwa 0,3 L Lösemittel Verwendung finden. Gegebenenfalls kann der Waschschritt wiederholt durchgeführt werden.
Das erhaltene Produkt wird im Vakuum oder mittels erwärmter Umluft bis zum Erreichen eines Wassergehalts von 2,5 - 4,0 % getrocknet.

Ein Aspekt der vorliegenden Erfindung betrifft kristallines Tiotropiumbromid-monohydrat, welches gemäß vorstehend beschriebener Vorgehensweise erhältlich ist.

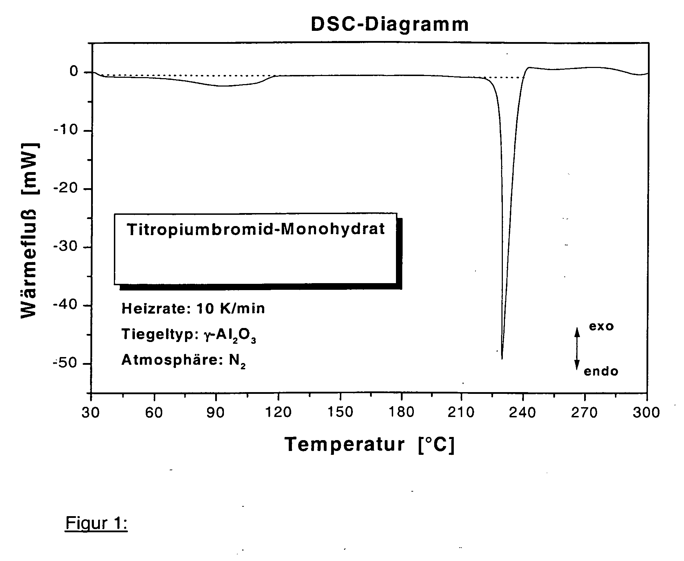

Das gemäß vorstehend beschriebener Vorgehensweise erhältliche Tiotropiumbromid-monohydrat wurde einer Untersuchung mittels DSC (Differential Scanning Calorimetry) unterworfen. Das DSC- diagramm weist zwei charakteristische Signale auf. Das erste, relativ breite, endotherme Signal zwischen 50-120°C ist auf die Entwässerung des Tiotropiumbomid-monohydrats zur wasserfreien Form zurückzuführen. Das zweite, relativ scharfe, endotherme Maximum bei 230 ± 5°C ist dem Schmelzen der Substanz zuzuordnen (Figur 1). Diese Daten wurden mittels eines Mettler DSC 821 erhalten und mit dem Mettler Software-Paket STAR ausgewertet. Die Daten wurden bei einer Heizrate von 10 K/min erhoben.
Da die Substanz unter Zersetzung schmilzt (= inkongruenter Schmelzvorgang), hängt der beobachtete Schmelzpunkt sehr von der Heizrate ab. Bei geringeren Heizraten wird der Schmelz-/Zersetzungsvorgang bei deutlich niedrigeren Temperaturen beobachtet, so zum Beispiel mit einer Heizrate von 3 K/min bei 220 ± 5 °C. Es kann außerdem vorkommen, daß der Schmelzpeak aufgespalten vorliegt. Die Aufspaltung tritt umso stärker auf, je geringer die Heizrate im DSC-Experiment ist. Dementsprechend zielt die vorliegende Erfindung auf kristallines Tiotropiumbromid-monohydrat, welches entsprechend Firgur 1 durch ein endothermes Maximum bei 230°C (± 5°C) bei einer Heizrate von 10K/min gekennzeichnet ist.

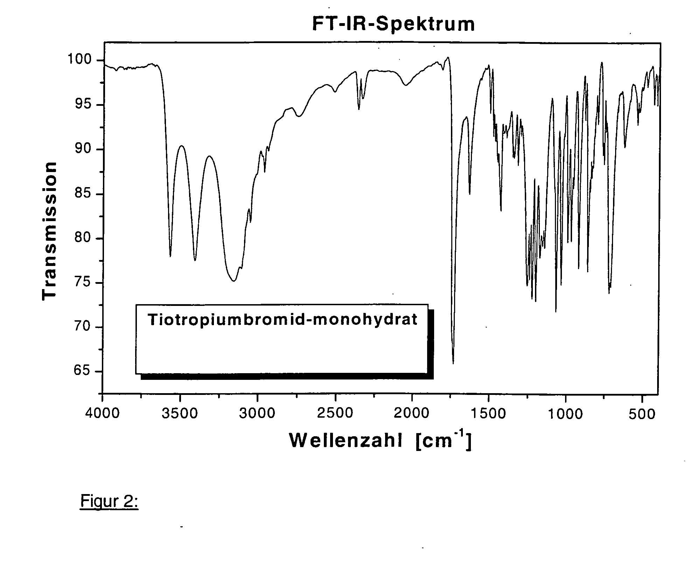

Das erfindungsgemäße Tiotropiumbromid-monohydrat wurde mittels IR-Spektroskopie charakterisiert. Die Daten wurden mittels eines Nicolet FTIR Spektrometers erhoben und mit dem Nicolet Softwarepaket OMNIC, Version 3.1 ausgewertet. Die Messung wurde mit 2,5µmol Tiotropiumbromid-monohydrat in 300 mg KBr durchgeführt. Das erhaltene IR-Spektrum ist in Figur 2 wiedergegeben. Tabelle 1 faßt einige der wesentlichen Banden des IR-Spektrums zusammen.

**Tabelle 1:**

| Zuordnung von spezifischen Banden | | |
|---|---|---|
| Wellenzahl (cm⁻¹) | Zuordnung | Schwingungstyp |
| 3570, 3410 | O-H | Streckschwingung |
| 3105 | Aryl C-H | Streckschwingung |
| 1730 | C=O | Streckschwingung |
| 1260 | Epoxid C-O | Streckschwingung |
| 1035 | Ester C-OC | Streckschwingung |
| 720 | Thiophen | Ringschwingung |

Dementsprechend betrifft die vorliegende Erfindung kristallines Tiotropiumbromid-monohydrat, welches entsprechend Firgur 2 durch ein IR-Spektrum gekennzeichnet ist, das unter anderen bei den Wellenzahlen 3570, 3410, 3105, 1730, 1260, 1035 und 720 cm⁻¹ Banden aufweist.

Das erfindungsgemäße Tiotropiumbromid-monohydrat wurde mittels Röntgenstrukturanalyse charakterisiert. Die Röntgenbeugungsintensitätsmessungen wurden auf einem AFC7R- 4-Kreisdiffraktometer (Rigaku) unter Verwendung von monochromatisierter Kupfer Kα-Strahlung durchgeführt. Die Strukturlösung und Verfeinerung der Kristallstruktur erfolgte mittels direkter Methoden (Programm SHELXS86) und FMLQ-Vefeinerung (Programm TeXsan). Experimentelle Details zur Kristallstruktur, Strukturlösung und -verfeinerung sind in Tabelle 2 zusammengefaßt.

**Tabelle 2:**

| Experimentelle Daten zur Kristallstrukturanalyse von Tiotropiumbromidmonohydrat. | |
|---|---|
| A. Kristalldaten | |
| Empirische Formel | [C₁₉H₂₂NO₄S₂] Br · H₂0 |
| Formelgewicht | 472.43 + 18.00 |
| Kristallfarbe, -gestalt | farblos, prismatisch |
| Kristallabmessungen | 0.2 x 0.3 x 0.3 mm |
| Kristallsystem | monoklin |
| Gittertyp | primitv |
| Raumgruppe | P 2₁/n |
| Gitterkonstanten | a = 18.0774 Å, b = 11.9711 Å c = 9.9321 Å β = 102.691° V = 2096.96 Å³ |
| Formeleinheiten pro Elementarzelle | 4 |

| B. Intensitätsmessungen | |
|---|---|
| Diffraktometer | Rigaku AFC7R |
| Röntgengenerator | Rigaku RU200 |
| Wellenlänge | λ= 1.54178Å (monochromatisierte Kupfer Kα-Strahlung) |
| Strom, Spannung | 50kV, 100mA |
| Take-off Winkel | 6° |
| Kristallmontage | wasserdampfgesättigte Kapillare |
| Kristall-Detektor-Abstand | 235mm |
| Detektor Öffnung | 3.0 mm vertikal und horizontal |
| Temperatur | 18° |
| Bestimmung der Gitterkonstanten | 25 Reflexe (50.8° < 2⊖ < 56.2°) |
| Scan Typ | ω - 2⊖ |
| Scan Geschwindigkeit | 8.0 32.0°/min in ω |
| Scan Breite | (0.58 + 0.30 tan ⊖) ° |
| 2⊖max | 120° |
| Messungen | 5193 |
| Unabhängige Reflexe | 3281 ( Rint=0.051) |
| Korrrektu ren | Lorentz-Polarisation Absorption (Transmissionsfaktoren 0.56 - 1.00) Kristall-decay 10.47% Abfall |

| C. Verfeinerung | |
|---|---|
| Reflexe (I > 3σI) | 1978 |
| Variable | 254 |
| Verhältnis Reflexe/Parameter | 7.8 |
| R-Werte: R, Rw | 0.062, 0.066 |

Die durchgeführte Röntgenstrukturanyluse ergab, daß kristallines Toptropiumbromidhydrat eine einfache monoklinische Zelle mit folgenden Dimensionen aufweist:
a = 18.0774 Å, b = 11.9711 Å, c = 9.9321 Å, β = 102.691 °, V = 2096.96 Å³.
Dementsprechend betrifft die vorliegende Erfindung kristallines Tiotropiumbromid-monohydrat, welches durch vorstehend beschriebene Elementarzelle gekennzeichnet ist.
Durch die vorstehende Röntgenstrukturanalyse wurden die in Tabelle 3 beschriebenen Atomkoordinaten bestimmt:

**Tabelle 3:**

| Koordinaten | | | | |
|---|---|---|---|---|
| Atom | x | y | z | u (eq) |
| Br(1) | 0.63938(7) | 0.0490(1) | 0.2651(1) | 0.0696(4) |
| S(1) | 0.2807(2) | 0.8774(3) | 0.1219(3) | 0.086(1) |
| S(2) | 0.4555(3) | 0.6370(4) | 0.4214(5) | 0.141 (2) |
| O(1) | 0.2185(4) | 0.7372(6) | 0.4365(8) | 0.079(3) |
| O(2) | 0.3162(4) | 0.6363(8) | 0.5349(9) | 0.106(3) |
| O(3) | 0.3188(4) | 0.9012(5) | 0.4097(6) | 0.058(2) |
| O(4) | 0.0416(4) | 0.9429(6) | 0.3390(8) | 0.085(3) |
| O(5) | 0.8185(5) | 0.0004(8) | 0.2629(9) | 0.106(3) |
| N(1 ) | 0.0111(4) | 0.7607(6) | 0.4752(7) | 0.052(2) |
| C(1) | 0.2895(5) | 0.7107(9) | 0.4632(9) | 0.048(3) |
| C(2) | 0.3330(5) | 0.7876(8) | 0.3826(8) | 0.048(3) |
| C(3) | 0.3004(5) | 0.7672(8) | 0.2296(8) | 0.046(3) |
| C(4) | 0.4173(5) | 0.7650(8) | 0.4148(8) | 0.052(3) |
| C(5) | 0.1635(5) | 0.6746(9) | 0.497(1) | 0.062(3) |
| C(6) | 0.1435(5) | 0.7488(9) | 0.6085(9) | 0.057(3) |
| C(7) | 0.0989(6) | 0.6415(8) | 0.378(1) | 0.059(3) |
| C(8) | 0.0382(5) | 0.7325(9) | 0.3439(9) | 0.056(3) |
| C(9) | 0.0761(6) | 0.840(1) | 0.315(1) | 0.064(3) |
| C(10) | 0.1014(6) | 0.8974(8) | 0.443(1) | 0.060(3) |
| C(11) | 0.0785(5) | 0.8286(8) | 0.5540(9) | 0.053(3) |
| C(12) | -0.0632(6) | 0.826(1) | 0.444(1) | 0.086(4) |
| C(13) | -0.0063(6) | 0.6595(9) | 0.554(1) | 0.062(3) |
| C(14) | 0.4747(4) | 0.8652(9) | 0.430(1) | 0.030(2) |
| C(15) | 0.2839(5) | 0.6644(9) | 0.1629(9) | 0.055(3) |
| C(16) | 0.528(2) | 0.818(2) | 0.445(2) | 0.22(1) |
| C(17) | 0.5445(5) | 0.702(2) | 0.441(1) | 0.144(6) |
| C(18) | 0.2552(6) | 0.684(1) | 0.019(1) | 0.079(4) |
| C(19) | 0.2507(6) | 0.792(1) | -0.016(1) | 0.080(4) |
| H(1) | -0.0767 | 0.8453 | 0.5286 | 0.102 |
| H(2) | -0.0572 | 0.8919 | 0.3949 | 0.102 |
| H(3) | -0.1021 | 0.7810 | 0.3906 | 0.102 |
| H(4) | -0.0210 | 0.6826 | 0.6359 | 0.073 |
| H(5) | -0.0463 | 0.6178 | 0.4982 | 0.073 |
| H(6) | 0.0377 | 0.6134 | 0.5781 | 0.073 |
| H(7) | 0.1300 | 0.7026 | 0.6770 | 0.069 |
| H(8) | 0.1873 | 0.7915 | 0.6490 | 0.069 |
| H(9) | 0.1190 | 0.6284 | 0.2985 | 0.069 |
| H(10) | 0.0762 | 0.5750 | 0.4016 | 0.069 |
| H(11) | 0.1873 | 0.6082 | 0.5393 | 0.073 |
| H(12) | -0.0025 | 0.7116 | 0.2699 | 0.066 |
| H(13) | 0.1084 | 0.8383 | 0.2506 | 0.075 |
| H(14) | 0.1498 | 0.9329 | 0.4626 | 0.071 |
| H(15) | 0.0658 | 0.8734 | 0.6250 | 0.063 |
| H(16) | 0.2906 | 0.5927 | 0.2065 | 0.065 |
| H(17) | 0.2406 | 0.6258 | -0.0469 | 0.094 |
| H(18) | 0.2328 | 0.8191 | -0.1075 | 0.097 |
| H(19) | 0.4649 | 0.9443 | 0.4254 | 0.037 |
| H(20) | 0.5729 | 0.8656 | 0.4660 | 0.268 |
| H(21) | 0.5930 | 0.6651 | 0.4477 | 0.165 |
| H(22) | 0.8192 | -0.0610 | 0.1619 | 0.084 |
| H(23) | 0.7603 | 0.0105 | 0.2412 | 0.084 |
| x, y, z: fraktionelle Koordinaten; | | | | |
| U(eq) mittlere quadratische Amplitude atomarer Bewegung im Kristall; | | | | |

Ein weiterer Aspekt der vorliegenden Erfindung betrifft aufgrund der pharmazeutischen Wirksamkeit des erfindungsgemäßen Hydrats die Verwendung von Tiotropiumbromid-monohydrat als Arzneimittel.
Zur Herstellung eines inhalativ applizierbaren Arzneimittels, insbesondere eines Inhalationspulvers, welches das durch die vorliegende Erfindung beschriebene kristalline Tiotropiumbromid-monohydrat enthält, kann nach im Stand der Technik bekannten Verfahren vorgegangen werden. Diesbezüglich wird beispielsweise auf die Lehre der DE-A-179 22 07 verwiesen. Dementsprechend zielt ein weiterer Aspekt der vorliegenden Erfindung auf Inhaltionspulver gekennzeichnet durch einen Gehalt von Tiotropiumbromid-monohydrat.
Aufgrund der anticholinergen Wirksamkeit von Tiotropiumbromid-monohydrat zielt ein weiterer Aspekt der vorliegenden Erfindung auf die Verwendung von Tiotropiumbromid-monohydrat zur Herstellung eines Arzneimittels zur Behandlung von Erkrankungen, in denen die Applikation eines Anticholinergikums einen therapeutischen Nutzen entfalten kann. Bevorzugt ist die entsprechende Verwendung zur Herstellung eines Arzneimittels zur Behandlung von Asthma oder COPD.

Das nachfolgende Synthesebeispiel dient der Illustration eines exemplarisch durchgeführter Herstellungsverfahrens für kristallines Tiotropiumbromid-monohydrat. Es ist lediglich als mögliche, exemplarisch dargestellte Vorgehensweise zu verstehen, ohne die Erfindung auf dessen Inhalt zu beschränken.

### Synthesebeispiel

In einem geeigneten Reaktionsgefäß werden in 25,7 kg Wasser 15,0 kg Tiotropiumbromid eingetragen. Die Mischung wird auf 80-90°C erhitzt und bei gleichbleibender Temperatur solange gerührt, bis eine klare Lösung entsteht. Aktivkohle (0,8 kg), wasserfeucht, wird in 4,4 kg Wasser aufgeschlämmt, diese Mischung in die Tiotropiumbromid-haltige Lösung eingetragen und mit 4,3 kg Wasser nachgespült. Die so erhaltene Mischung wird wenigstens 15 min bei 80-90°C gerührt und anschließend über einen beheizten Filter in einen auf 70°C Manteltemperatur vorgewärmten Apparat filtriert. Der Filter wird mit 8,6 kg Wasser nachgespült. Der Apparateinhalt wird mit 3-5°C pro 20 Minuten auf eine Temperatur von 20-25°C abgekühlt. Mit Kaltwasserkühlung wird der Apparat auf 10-15°C weiter abgekühlt und die Kristallisation durch mindestens einstündiges Nachrühren vervollständigt. Das Kristallisat wird über einen Nutschentrockner isoliert, der isolierte Kristallbrei mit 9 L kaltem Wasser (10-15°C) und kaltem Aceton (10-15°C) gewaschen. Die erhaltenen Kristalle werden bei 25°C über 2 Stunden im Stickstoffstrom getrocknet.
Ausbeute : 13,4 kg Tiotropiumbromid-monohydrat (86 % d. Th.)

## Patentansprüche

1. Kristallines Tiotropiumbromid-Monohydrat.

2. Kristallines Tiotropiumbromid-Monohydrat nach Anspruch 1, **gekennzeichnet durch** ein bei der thermischen Analyse mittels DSC auftretendes endothermes Maximum bei 230 ± 5°C bei einer Heizrate von 10K/min.

3. Kristallines Tiotropiumbromid-Monohydrat nach Anspruch 1 oder 2, **gekennzeichnet durch** ein IR-Spektrum, das unter anderen bei den Wellenzahlen 3570, 3410, 3105, 1730, 1260, 1035 und 720 cm⁻¹ Banden aufweist.

4. Kristallines Tiotropiumbromid-Monohydrat nach einem der Ansprüche 1, 2 oder 3, **gekennzeichnet durch** eine einfache monoklinische Zelle mit folgenden Dimensionen: a = 18.0774 Ä, b = 11.9711 Å, c = 9.9321 Å, β = 102.691 °, V = 2096.96 Å³.

5. Verfahren zur Herstellung von kristallinem Tiotropiumbromid-monohydrat gemäß einem der Ansprüche 1, 2, 3 oder 4, **dadurch gekennzeichnet, daß**
a) Tiotropiumbromid in Wasser aufgenommen wird,
b) die erhaltene Mischung erwärmt wird,
c) Aktivkohle zugesetzt wird und
d) nach Abtrennen der Aktivkohle Tiotropiumbromid-monohydrat unter langsamem Abkühlen der wässrigen Lösung langsam kristallisiert wird.

6. Verfahren gemäß Anspruch 5, **dadurch gekennzeichnet, daß**
a) pro Mol eingesetztes Tiotropiumbromid 0,4 bis 1,5 kg Wasser verwendet werden,
b) die erhaltene Mischung auf mehr als 50°C erwärmt wird,
c) pro Mol eingesetztes Tiotropiumbromid 10 bis 50 g Aktivkohle eingesetzt werden und nach erfolgter Aktivkohlezugabe zwischen 5 bis 60 Minuten, weitergerührt wird,
d) die erhaltene Mischung filtriert, das erhaltene Filtrat mit einer Abklühlrate von 1 bis 10°C pro 10 bis 30 Minuten auf eine Temperatur von 20-25°C abgekühlt und das Tiotropiumbromid-monohydrat dabei kristallisiert wird.

7. Arzneimittel **gekennzeichnet durch** einen Gehalt an kristallinem Tiotropiumbromid-monohydrat nach einem der Ansprüche 1 bis 4.

8. Arzneimittel nach Anspruch 6, **dadurch gekennzeichnet, daß** es sich um ein Inhalationspulver handelt.

9. Verwendung von kristallinem Tiotropiumbromid-monohydrat nach einem der Ansprüche 1 bis 4 zur Herstellung eines Arzneimittels zur Behandlung von Erkrankungen, in denen die Applikation eines Anticholinergikums einen therapeutischen Nutzen entfalten kann.

10. Verwendung nach Anspruch 9, **dadurch gekennzeichnet, daß** es sich bei den Erkrankungen um Asthma oder COPD handelt.

11. Verfahren zur Herstellung von kristallinen Hydraten des Tiotropiumbromids, **dadurch gekennzeichnet, daß** Tiotropiumbromid in Wasser aufgenommen wird, die erhaltene Mischung erwärmt wird und abschließend die Hydrate des Tiotropiumbromids unter langsamem Abkühlen kristallisiert werden.

12. Kristalline Hydrate des Tiotropiumbromids, erhältlich durch das Verfahren nach Anspruch 11.
